# FASCICULE DE BREVET EUROPEEN

(11) **EP 2 502 577 B1**
(45) Date de publication et mention de la délivrance du brevet: **02.11.2016**
(21) Numéro de dépôt: 12158011.2
(22) Date de dépôt: 05.03.2012
(51) Int. Cl.: A61B 17/11, A61F 2/06, A61M 39/10, A61M 1/10

(54) **Prothèse pour assurer le raccordement d'un canal anatomique.**
Prothese zur Sicherstellung eines Anschlusses an einen anatomischen Kanal.
Prosthesis for connecting an anatomical canal

(30) Priorité: 22.03.2011 FR 1152364
(43) Date de publication de la demande: 26.09.2012
(73) Titulaire: CARMAT, 78941 Vélizy Villacoublay cedex (FR)
(72) Inventeur: Da Cruz Louro, Pierre, 91100 Villabe (FR)
(74) Mandataire: Gevers & Orès

(56) Documents cités:
- WO-A1-00/24339
- WO-A1-03/086528
- WO-A1-2004/001272
- WO-A1-2010/022705
- US-A1- 2004 054 405
- US-A1- 2010 036 397

## Description

La présente invention concerne une prothèse pour assurer le raccordement d'un canal anatomique.

Bien qu'elle soit particulièrement adaptée au raccordement de deux portions d'un même canal anatomique (par exemple un vaisseau sanguin), la prothèse de la présente invention peut également être mise en oeuvre pour raccorder un canal anatomique à un organe ou à un dispositif artificiel (par exemple une prothèse cardiaque ou une assistance ventriculaire).

Par ailleurs, on sait que certaines maladies ou certains troubles médicaux nécessitent l'élimination d'une partie d'un vaisseau sanguin (par exemple une artère ou une veine) et son remplacement par une prothèse vasculaire. Une telle prothèse comporte, par exemple, un corps tubulaire monolithique formant un canal anatomique sur toute sa longueur, généralement de forme cylindrique.

On sait également que, pour implanter une prothèse vasculaire du type précité, les équipes chirurgicales doivent couper la portion malade du vaisseau, pour ensuite suturer chacune des parties libres du vaisseau ainsi coupé à une extrémité de la prothèse vasculaire monolithique.

Cependant, du fait que certains vaisseaux sont difficiles d'accès et/ou du fait que la suture d'une prothèse à un vaisseau est difficile à réaliser, les chirurgiens sont contraints de couper en deux la prothèse vasculaire, de manière à faciliter la suture des extrémités de ladite prothèse au vaisseau à traiter. Une fois celle-ci réalisée, les chirurgiens suturent les deux extrémités libres de la prothèse, l'une à l'autre.

Autrement dit, une telle prothèse vasculaire requiert une zone de suture supplémentaire (celle définie entre les deux extrémités coupées de la prothèse), ce qui augmente le temps d'implantation de la prothèse ainsi que les risques hémodynamiques (par exemple stase, hémorragie par les points de suture effectués, etc...).

De tels inconvénients sont également observés lors du raccordement d'un vaisseau sanguin à une prothèse cardiaque, par l'intermédiaire d'une prothèse vasculaire monolithique du type précité.

Des moyens pour assurer le raccordement d'un canal anatomique sont connus des documents WO-A-2004/001272 et WO-A-00/24339.

La présente invention a pour objet de remédier à ces inconvénients.

A cette fin, selon l'invention, la prothèse tubulaire pour le raccordement d'un canal anatomique comporte au moins deux éléments tubulaires individuels et des moyens mécaniques pour raccorder lesdits éléments individuels de prothèse l'un à l'autre, et les dits moyens mécaniques de raccord comportent:
- deux pièces de jonction en forme de couronne, chacune d'elles étant fixée à l'extrémité libre d'un élément tubulaire individuel ;
- au moins un joint d'étanchéité (se présentant, par exemple, sous la forme un joint mécanique compressible préformé ou bien encore d'une colle chirurgicale G.R.F (Gélatine - Résorcinol - Formaldéhyde)) porté par une des deux pièces de jonction et destiné à être intercalé entre les deux pièces de jonction autour de la lumière formée par la prothèse tubulaire; et
- une première et une seconde bagues de fixation traversées chacune par un élément tubulaire individuel et destinées à coopérer l'une avec l'autre pour maintenir ensemble, l'une contre l'autre, les deux pièces de jonction entre lesquelles est comprimé le joint d'étanchéité.

Ainsi, grâce à l'invention, il n'est plus nécessaire de couper la prothèse, celle-ci étant formée d'au moins deux éléments tubulaires individuels, ce qui garantit une zone de coupure propre et régulière. En outre, les moyens mécaniques de raccord, montés sur la prothèse, permettent de joindre ensemble, rapidement et de façon aisée, les deux extrémités libres des éléments individuels, sans réalisation de suture supplémentaire. En conséquence, les risques hémodynamiques sont sensiblement réduits et le travail des chirurgiens notablement facilité.

De plus, les premières et secondes bagues de fixation assurent la tenue des pièces de jonction l'une contre l'autre et la compression du joint d'étanchéité entre celles-ci, ce qui permet de garantir l'étanchéité le long de la prothèse tubulaire (notamment au niveau de la jonction des deux pièces). On évite en outre que du fluide corporel (par exemple du sang lorsque le canal anatomique est un vaisseau sanguin), qui s'écoule dans la prothèse, ne vienne au contact de matériaux autres que celui des éléments tubulaires individuels (qui sont de préférence réalisés en matière biocompatible, telle qu'un polyester tissé).

Dans une forme de réalisation conforme à l'invention :
- la première bague de fixation comprend une collerette interne sur laquelle est destinée à être plaquée une des couronnes ;
- la seconde bague de fixation comprend un collier annulaire saillant au bout duquel est montée solidaire l'autre couronne ; et
- le collier saillant est apte à être introduit à l'intérieur de la première bague, afin de mettre en contact les deux couronnes l'une avec l'autre.

En outre, selon cette forme de réalisation :
- la première bague de fixation peut comporter des saillies radiales internes ; et
- la seconde bague de fixation peut comprendre des logements ménagés dans la paroi latérale du collier saillant et aptes à recevoir les saillies radiales correspondantes de la première bague.

De plus, chacune des saillies radiales peut se présenter sous la forme d'un tenon et chacun des logements peut comporter :
- une encoche axiale permettant l'introduction d'une saillie radiale ; et
- une rampe de verrouillage s'étendant sur un secteur angulaire déterminé et dont l'entrée est formée par l'encoche axiale associée.

En particulier, la rampe de verrouillage peut être hélicoïdale et se terminer par une encoche de verrouillage, ce qui permet de compresser le joint d'étanchéité de façon prédéfinie et de le maintenir dans cette position de verrouillage.

De surcroît, les saillies radiales et les logements correspondants, de préférence au nombre de trois, peuvent être équi-angulairement répartis, afin de maintenir une compression uniforme du joint d'étanchéité, le long de celui-ci.

Dans une variante de la forme de réalisation conforme à l'invention, la paroi latérale interne de la première bague présente un filetage apte à coopérer avec un filetage correspondant pratiqué dans la paroi latérale externe du collier de fixation. En outre, la première bague, montée libre en rotation autour de l'élément tubulaire individuel correspondant, est indépendante de la couronne associée à ce dernier.

Par ailleurs, la prothèse comporte avantageusement des moyens de fixation auxiliaires, pour sécuriser le maintien desdites couronnes l'une contre l'autre.

En particulier, ces moyens de fixation auxiliaires peuvent comporter au moins un oeillet de fixation, monté solidaire de la première bague de fixation, et au moins un orifice de fixation correspondant, ménagé dans la paroi latérale de la seconde bague de fixation, l'oeillet et l'orifice de fixation étant aptes à être reliés l'un à l'autre, par exemple, par un fil de suture.

Par ailleurs, on remarquera que, en fonction de la forme du canal anatomique, la prothèse peut présenter une forme générale cylindrique, conique, bifurquée (lorsque le canal anatomique est lui-même bifurquée), etc ...
Les figures du dessin annexé feront bien comprendre comment l'invention peut être réalisée. Sur ces figures, des références identiques désignent des éléments semblables.
La figure 1 représente schématiquement, dans une vue en perspective, un exemple de réalisation de la prothèse conforme à la présente invention, dans laquelle les deux éléments tubulaires individuels ne sont pas raccordés l'un à l'autre.
La figure 2 est semblable à la figure 1, à l'exception du fait que les deux éléments tubulaires individuels de la prothèse sont raccordés l'un à l'autre.
Les figures 3 et 4 sont des vues schématiques de face d'une extrémité libre d'un élément tubulaire individuel comportant respectivement la première bague de fixation (figure 3) et la seconde bague de fixation (figure 4).
La figure 5 est une coupe schématique longitudinale de la prothèse de l'invention, selon la ligne de coupe V-V des figures 3 et 4, lorsque les deux éléments tubulaires individuels de la prothèse ne sont pas raccordés l'un à l'autre.
La figure 6 est semblable à la figure 5, excepté par le fait que les deux éléments tubulaires individuels sont désormais raccordés l'un à l'autre.
La figure 7 représente, de façon schématique, un agrandissement de la zone A de la figure 6, lorsque les deux éléments tubulaires individuels ne sont pas raccordés l'un à l'autre.
La figure 8 est un agrandissement schématique de la zone B de la figure 6.

Comme mentionné précédemment, la prothèse conforme à la présente invention peut être utilisée tout aussi bien pour le raccordement de deux portions d'un même canal anatomique (par exemple un vaisseau sanguin) que pour le raccordement d'un canal anatomique à un organe ou à un dispositif artificiel (par exemple un coeur artificiel).

Pour des raisons de clarté et de concision, la prothèse de l'invention sera plus particulièrement décrite ci-après en rapport avec les vaisseaux sanguins.

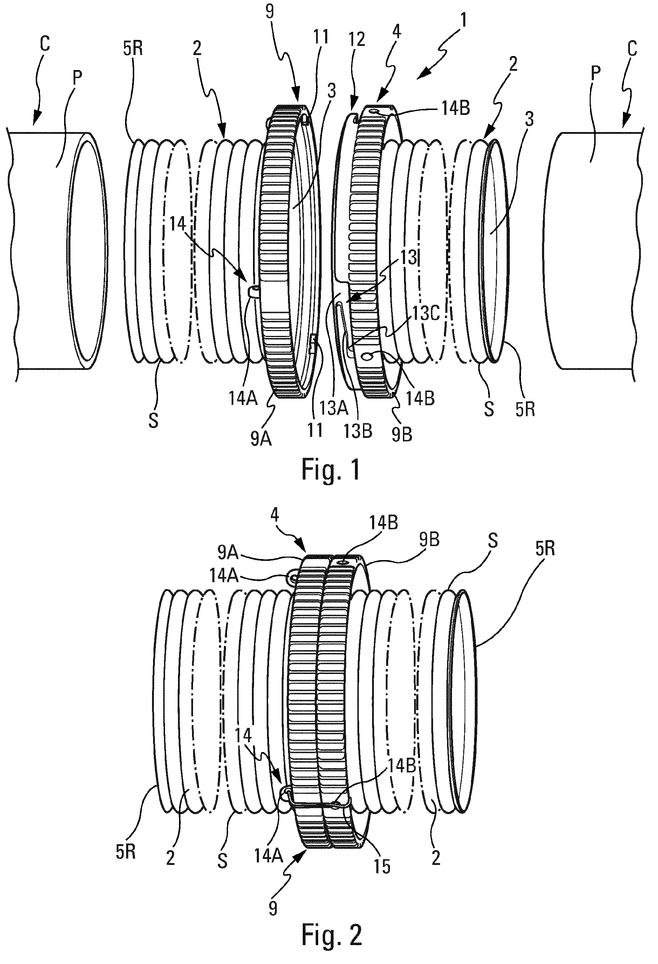

La prothèse tubulaire 1, conforme à la présente invention et illustrée par les figures 1 et 2, comprend deux éléments tubulaires individuels 2, par exemple formé en polyester tissé, définissant chacun une lumière interne 3.

Les éléments tubulaires individuels 2, de forme cylindrique, sont destinés à être suturés, par un chirurgien, à l'une de leurs extrémités 5R, à une portion P d'un canal anatomique C à traiter.

La surface externe S de chacun des éléments tubulaires individuels 2 est annelée pour être souple et pour assurer une meilleure prise dans la portion P correspondante du canal anatomique C.

La prothèse 1 comprend en outre des moyens mécaniques étanches 4 pour raccorder les extrémités libres respectives 5L des deux éléments tubulaires individuels 2 (les extrémités libres 5L étant celles qui ne sont pas suturées aux portions P).

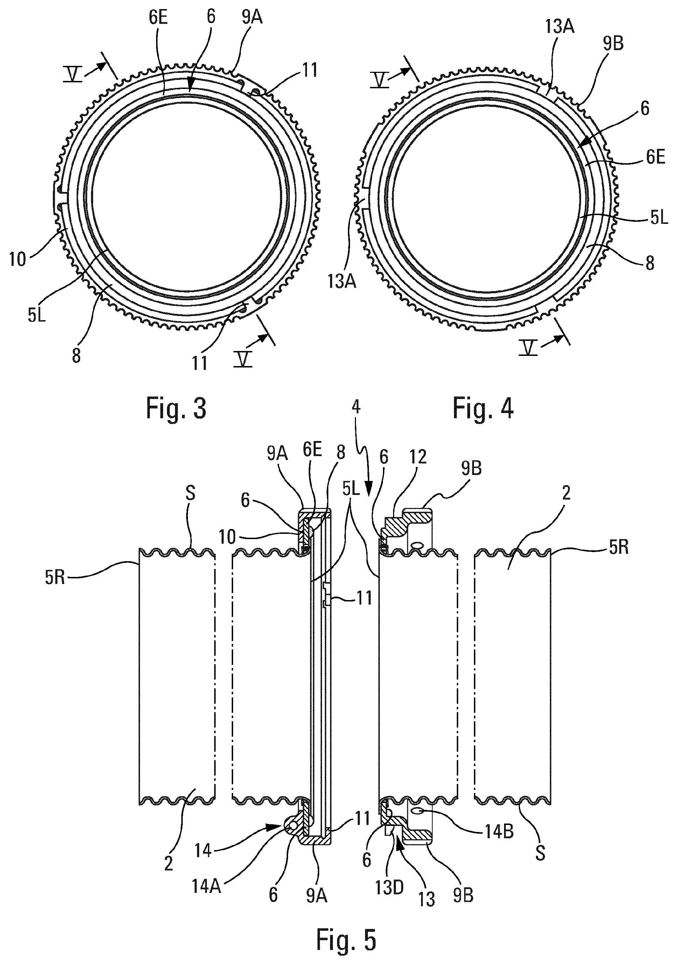

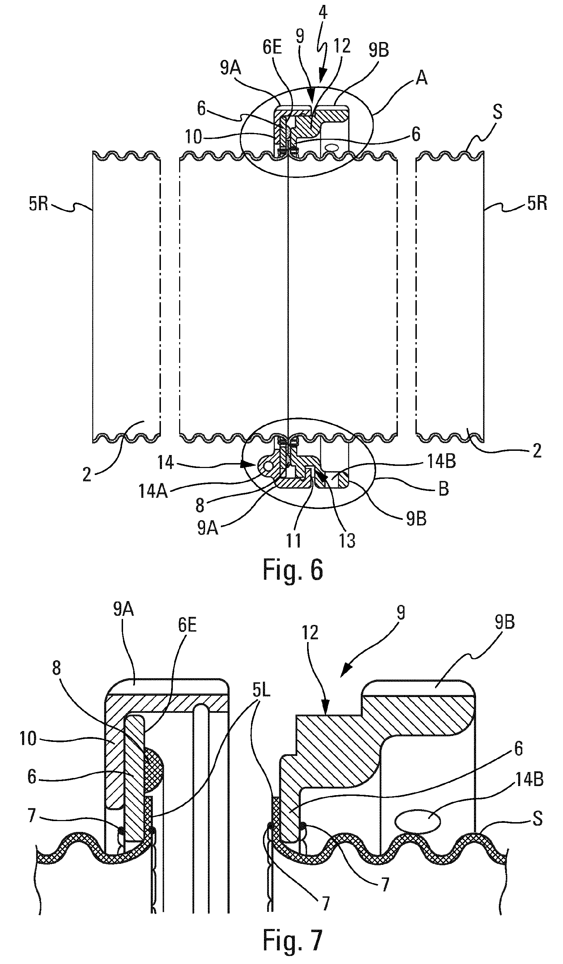

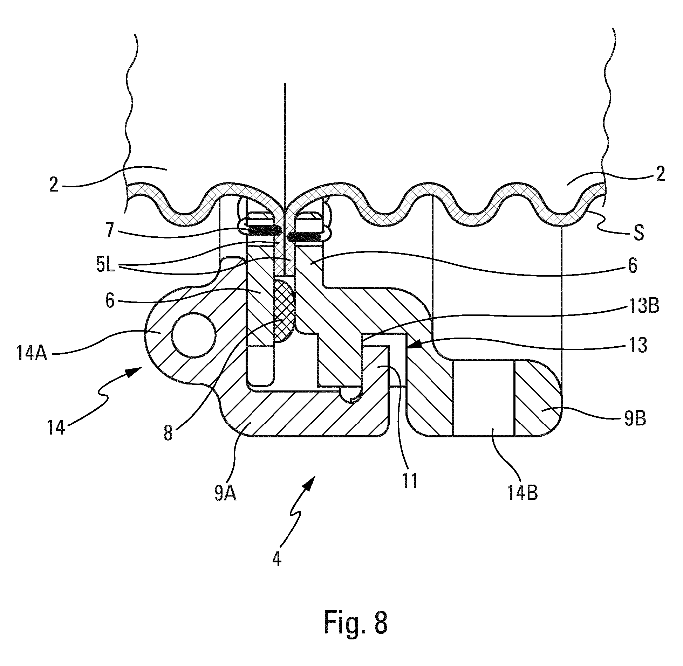

Comme le montrent les figures 3 à 8, les moyens mécaniques de raccord 4 comportent :
- deux pièces de jonction 6, en forme de couronne, qui sont fixées respectivement à une extrémité libre 5L d'un élément tubulaire individuel 2. Chaque élément tubulaire 2 peut être fixé à une couronne 6 par collage et/ou par suture 7 de son extrémité libre 5L à celle-ci (la suture pouvant être rendue étanche lors du procédé de fabrication de la prothèse 1). En particulier, chaque élément tubulaire 2 traverse la lumière interne de la couronne 6 correspondante, afin venir recouvrir, par son extrémité libre 5L, au moins une partie de la surface extérieure 6E (c'est-à-dire tournée vers l'autre élément tubulaire 2) de cette dernière;
- un joint d'étanchéité 8 annulaire, qui est solidaire de la surface extérieure 6E d'une couronne 6 et qui est agencé au voisinage de la lumière 3. Le joint 8 est destiné à être comprimé par les deux couronnes 6, lorsque les deux éléments tubulaires individuels 2 correspondants sont raccordés l'un à l'autre ; et
- des moyens mécaniques de maintien 9 des couronnes 6, l'une contre l'autre, tout en compressant entre elles le joint d'étanchéité 8 de manière à assurer l'étanchéité le long de la prothèse tubulaire 1.

Les moyens 9 de maintien comportent une première 9A et une seconde 9B bagues de fixation, qui sont destinées à coopérer l'une avec l'autre et qui sont traversées chacune par l'élément tubulaire 2 associé.

Dans cet exemple, la première bague 9A comprend une collerette interne 10 sur laquelle une couronne 6 correspondante peut venir se plaquée. Elle comporte en outre des saillies 11, en forme de tenon, qui s'étendent radialement vers l'intérieur de la bague 9A.

La seconde bague 9B comprend un collier annulaire saillant 12 au bout duquel est montée solidaire l'autre couronne 6. Avantageusement, la bague 9B, le collier 12 et la couronne 6 associée peuvent être formés en une seule et même pièce.

Le collier saillant 12 est apte à être introduit, avec ajustement, à l'intérieur de la première bague 9A. Ainsi, la couronne 6 solidaire du collier saillant 12 est plaquée contre le joint d'étanchéité 8 porté par la couronne 6 de la bague 9B.

En outre, le collier 12 comprend des logements 13, qui sont ménagés dans la paroi latérale de celui-ci et qui sont aptes à recevoir les saillies radiales 11 correspondantes.

Chaque logement 13 comporte :
- une encoche axiale 13A orientée vers l'extérieur et apte à recevoir un des tenons 11 ; et
- une rampe de verrouillage 13B s'étendant sur un secteur angulaire déterminé et dont l'entrée est formée par une encoche axiale 13A associée.

En particulier, chaque rampe de verrouillage 13B peut être hélicoïdale pour se terminer par une encoche de verrouillage 13C, ce qui permet de compresser le joint d'étanchéité de façon prédéfinie lorsque le tenon 11 correspondant a atteint l'encoche de verrouillage 13C tout en bloquant les deux bagues 9A et 9B l'une par rapport à l'autre, dans une position de verrouillage.

Dans l'exemple décrit, la première bague 9A comporte trois tenons 11 et, en conséquence, le collier 12 comprend trois logements 13 associés pour recevoir les tenons 11 correspondants.

Les tenons 11 ainsi que les logements 13 correspondants sont avantageusement équi-angulairement répartis, afin de maintenir une compression uniforme le long du joint 8.

Par ailleurs, pour sécuriser davantage le blocage des deux bagues l'une par rapport à l'autre, la prothèse 1 comporte des moyens de fixation auxiliaires 14.

Dans l'exemple décrit, les moyens de fixation auxiliaires 14 se présentent sous la forme d'oeillets de fixation 14A, montés solidaire de la première bague de fixation 9A, auxquels sont respectivement associés des orifices de fixation 14B, ménagés dans la paroi latérale de la seconde bague de fixation 9B à fond ouvert.

Pour bloquer les deux bagues 9A et 9B l'une par rapport à l'autre dans la position de verrouillage, du fil de suture 15 (voir la figure 2) peut par exemple être utilisé, de manière à relier chaque oeillet 14A à l'orifice de fixation 14B correspondant pour éviter que les bagues 9A et 9B ne tournent l'une par rapport à l'autre.

Chaque oeillet 14A est monté sur la première bague 9A dans un prolongement longitudinal d'un tenon 11. En outre, chaque orifice de fixation 14B est agencé dans un prolongement longitudinal d'une encoche de verrouillage 13C.

Ainsi, lorsque les tenons 11 occupent l'encoche 13C des logements 13, les oeillets 14A sont alignés longitudinalement avec les orifices de fixation 14B correspondants (voir notamment la figure 2).

Par ailleurs, il va de soi que la présente invention ne se limite pas à l'exemple de réalisation des moyens de mécaniques de raccord décrits ci-dessus.

## Revendications

1. Prothèse tubulaire de raccordement pour canal anatomique (C), qui comprend au moins deux éléments tubulaires individuels (2) et des moyens mécaniques (4) pour raccorder de façon étanche lesdits éléments individuels de prothèse l'un à l'autre, **caractérisée en ce que** lesdits moyens mécaniques de raccord étanches (4) comportent :
- deux pièces de jonction (6) en forme de couronne, chacune d'elles étant fixée à l'extrémité libre (5L) d'un élément tubulaire individuel (2) ;
- au moins un joint d'étanchéité (8) porté par une des deux pièces de jonction (6) et destiné à être intercalé entre les deux pièces de jonction (6) autour de la lumière (3) formée par la prothèse tubulaire ; et
- une première et seconde bagues de fixation (9A, 9B) traversées chacune par un élément tubulaire individuel (2) et destinées à coopérer l'une avec l'autre pour maintenir ensemble, l'une contre l'autre, les deux pièces de jonction (6) entre lesquelles est comprimé le joint d'étanchéité (8).

2. Prothèse selon la revendication 1,
**caractérisée en ce que** :
- la première bague de fixation (9A) comprend une collerette interne (10) sur laquelle est destinée à être plaquée une des couronnes (6) ;
- la seconde bague de fixation (9B) comprend un collier annulaire saillant (12) au bout duquel est montée solidaire l'autre couronne (6) ; et
- le collier saillant (12) est apte à être introduit à l'intérieur de la première bague (9A), afin de mettre en contact les deux couronnes (6) l'une avec l'autre.

3. Prothèse selon la revendication 2,
**caractérisée en ce que** :
- la première bague de fixation (9A) comporte des saillies radiales internes (11) ; et
- la seconde bague de fixation (9B) comprend des logements (13) ménagés dans la paroi latérale du collier saillant (12) et aptes à recevoir les saillies radiales (11) correspondantes de la première bague (9A).

4. Prothèse selon la revendication 3,
**caractérisée en ce que** :
- chacune des saillies radiales (11) se présente sous la forme d'un tenon; et
- chacun des logements (13) comporte :
▪ une encoche axiale (13A) permettant l'introduction d'une saillie radiale (11) ; et
▪ une rampe de verrouillage (13B) s'étendant sur un secteur angulaire déterminé et dont l'entrée est formée par une encoche axiale (13A) associée.

5. Prothèse selon la revendication 4,
**caractérisée en ce que** la rampe de verrouillage (13B) est hélicoïdale et se termine par une encoche de verrouillage (13C).

6. Prothèse selon l'une des revendications 3 à 5,
**caractérisée en ce que** les saillies radiales (11) et les logements (13) correspondants sont équi-angulairement répartis.

7. Prothèse selon l'une des revendications 1 à 6,
**caractérisée en ce qu'**elle comporte des moyens de fixation auxiliaires (14) pour sécuriser le maintien desdites couronnes (6) l'une contre l'autre.

8. Prothèse selon la revendication 7,
**caractérisée en ce que** lesdits moyens de fixation auxiliaires (14) comportent au moins un oeillet de fixation (14A), monté solidaire de la première bague de fixation (9A), et au moins un orifice de fixation (14B) correspondant, ménagé dans la paroi latérale de la seconde bague de fixation (9B).

## Patentansprüche

1. Röhrenprothese zum Anschließen für anatomischen Kanal (C), die mindestens zwei einzelne Röhrenelemente (2) und mechanische Mittel (4) zum dichten Anschließen der Protheseneinzelelemente aneinander umfasst, **dadurch gekennzeichnet, dass** die dichten mechanischen Anschlussmittel (4) Folgendes umfassen:
- zwei Verbindungsteile (6) in Kranzform, von welchen jedes an dem freien Ende (5L) eines einzelnen Röhrenelements (2) befestigt ist,
- mindestens eine Dichtung (8), die von einem der zwei Verbindungsteile (6) getragen wird und dazu bestimmt ist, zwischen die zwei Verbindungsteile (6) um das Langloch (3), das von der Röhrenprothese gebildet wird, eingefügt zu sein, und
- einen ersten und zweiten Befestigungsring (9A, 9B), die jeweils von einem einzelnen Röhrenelement (2) durchquert und dazu bestimmt sind, miteinander zusammenzuwirken, um die zwei Verbindungsteile (6), zwischen welchen die Dichtung (8) komprimiert ist, eines gegen das andere zusammenzuhalten.

2. Prothese nach Anspruch 1, **dadurch gekennzeichnet, dass**:
- der erste Befestigungsring (9A) einen internen Kragen (10) umfasst, auf den einer der Kränze (6) angedrückt werden soll,
- der zweite Befestigungsring (9B) einen ringförmigen vorstehenden Kragen (12) umfasst, an dessen Ende der andere Kranz (6) fest installiert ist, und
- der vorstehende Kragen (12) geeignet ist, in das Innere des ersten Rings (9A) eingeführt zu werden, um die zwei Kränze (6) miteinander in Berührung zu setzen.

3. Prothese nach Anspruch 2, **dadurch gekennzeichnet, dass**
- der erste Befestigungsring (9A) innere radiale Vorsprünge (11) umfasst, und
- der zweite Befestigungsring (9B) Aufnahmen (13) umfasst, die in der Seitenwand des vorstehenden Kragens (12) eingerichtet und geeignet sind, um die entsprechenden radialen Vorsprünge (11) des ersten Rings (9A) aufzunehmen.

4. Prothese nach Anspruch 3, **dadurch gekennzeichnet, dass**
- jeder der radialen Vorsprünge (11) die Form eines Zapfens hat, und
- jede der Aufnahmen (13) Folgendes umfasst:
• eine axiale Kerbe (13A), die das Einführen eines radialen Vorsprungs (11) erlaubt, und
• eine Verriegelungsrampe (13B), die sich auf einem bestimmten Winkelsektor erstreckt und deren Eingang von einer dazu gehörenden axialen Kerbe (13A) gebildet wird.

5. Prothese nach Anspruch 4, **dadurch gekennzeichnet, dass** die Verriegelungsrampe (13B) schraubenförmig ist und in einer Verriegelungskerbe (13C) endet.

6. Prothese nach einem der Ansprüche 3 bis 5, **dadurch gekennzeichnet, dass** die radialen Vorsprünge (11) und die entsprechenden Aufnahmen (13) gleichmäßig winkelig verteilt sind.

7. Prothese nach einem der Ansprüche 1 bis 6, **dadurch gekennzeichnet, dass** sie Hilfsbefestigungsmittel (14) umfasst, um das Halten der Kränze (6) gegeneinander abzusichern.

8. Prothese nach Anspruch 7, **dadurch gekennzeichnet, dass** die Hilfsbefestigungsmittel (14) mindestens eine Befestigungsöse (14A) umfassen, die fest verbunden mit dem ersten Befestigungsring (9A) installiert ist, und mindestens eine entsprechende Befestigungsöffnung (14B), die in der Seitenwand des zweiten Befestigungsrings (9B) ausgestaltet ist.

## Claims

1. Tubular prosthesis for connecting an anatomical duct (C), which comprises at least two individual tubular elements (2) and mechanical means (4) for connecting in a sealed manner said individual elements of the prosthesis to each other,
**characterised in that** said sealed mechanical connecting means (4) comprise:
- two annulus-shaped joining parts (6) each of them being fixed to the free end (5L) of an individual tubular element (2);
- at least one seal (8) carried by one of the two joining parts (6) and designed to be inserted between the two joining parts (6) around the opening (3) formed by the tubular prosthesis; and
- a first and a second fixing ring (9A, 9B) each traversed by an individual tubular element (2) and designed to cooperate with each other to hold together, one against the other, the two joining parts (6) between which the seal (8) is compressed.

2. Prosthesis according to claim 1,
**characterised in that**:
- the first fixing ring (9A) comprises an inner flange (10) on which one of the annuluses (6) is designed to be firmly pressed;
- the second fixing ring (9B) comprises a projecting annular collar (12) at the end of which the other annulus (6) is positively connected; and
- the projecting collar (12) is suitable for being inserted inside the first ring (9A) in order to put the two annuluses (6) in contact with each other.

3. Prosthesis according to claim 2,
**characterised in that**:
- the first fixing ring (9A) comprises internal radial projections (11); and
- the second fixing ring (9B) comprises recesses (13) made in the side wall of the projecting collar (12) and which are suitable for receiving the corresponding radial projections (11) of the first ring (9A).

4. Prosthesis according to claim 3,
**characterised in that**:
- each of the radial projections (11) is in the form of a stud; and
- each of the recesses (13) comprises:
• an axial notch (13A) for the insertion of a radial projection (11); and
• a locking incline (13B) extending over a particular angular sector, the entrance of which is formed by an associated axial notch (13A).

5. Prosthesis according to claim 4,
**characterised in that** the locking incline (13B) is helical and ends in a locking notch (13C).

6. Prosthesis according to any one of claims 3 to 5,
**characterised in that** the radial projections (11) and the corresponding recesses (13) are distributed at equal angles.

7. Prosthesis according to any one of claims 1 to 6,
**characterised in that** it comprises auxiliary fixing means (14) for holding said annuluses (6) securely against each other.

8. Prosthesis according to claim 7,
**characterised in that** said auxiliary fixing means (14) comprise at least one fixing eyelet (14A) positively connected to the first fixing ring (9A), and at least one corresponding fixing orifice (14B) made in the side wall of the second fixing ring (9B).
